# EUROPEAN PATENT SPECIFICATION

(11) **EP 0 967 968 B1**
(45) Date of publication and mention of the grant of the patent: **17.07.2002**
(21) Application number: 98904320.3
(22) Date of filing: 05.03.1998
(51) Int. Cl.: A61K 7/48

(54) **Pharmaceutical or cosmetic composition containing at least one retinoid and melibiose**
Mindestens ein Retinoid und Melibiose enthaltende pharmazeutische oder kosmetische Zusammensetzung
Composition pharmaceutique ou cosmetique contenant au moins un retinoide et melibiose

(30) Priority: 07.03.1997 FR 9702770
(43) Date of publication of application: 05.01.2000
(73) Proprietor: Johnson & Johnson Consumer France, 92130 Issy les Moulineaux (FR)
(72) Inventor: RIES, Gerd, D-40474 Düsseldorf (DE); CASTELLI, Dominique, F-75017 Paris (FR)
(74) Representative: Martin, Jean-Jacques
(86) International application number: IB9800274
(87) International publication number: WO9838980

(56) References cited:
- EP-A- 0 698 392
- WO-A-97/12597
- DATABASE "CHEMICAL ABSTRACTS" (HOST: STN); Abs.: 126: 297 580, Colombus, OH, USA; & M. DIAZ et al.:"Preformulation of vitamin A tablets" Rev. Cubana Farm. XP002067316
- DATABASE "CHEMICAL ABSTRACTS" (HOST: STN); Abs. 124: 156 108, Colombus, OH, USA; & JP 07 316 075 A(POLA KASEI K.K.K.) 5 MAY 1995 XP002067317

## Description

The present invention relates to pharmaceutical or cosmetological compositions designed to improve the appearance of the skin and to combat the effects of ageing of the skin.

The skin is composed of three compartments, the epidermis, including the outer layer and the stratum corneum, the dermis and, deeper down, the hypodermis. Exchanges exist between these different dermal and epidermal compartments, which are intended to ensure cell renewal, cohesion and moisturization of the outer layers.

It is known that ageing is a physiological phenomenon which follows a period of growth. It is reflected in particular by thinning of the skin and a loss of elasticity, leading to the appearance of deep or fine wrinkles; there is also surface drying and disorderly pigmentation may be observed.

All these signs reflect the changes in the epidermis, the epidermal junction and the dermis.

The dermis results from the biosynthetic activity of fibroblasts, which develop the constituents of the extracellular matrix; the latter is formed of four large families of macromolecules: collagens, elastin, structural glycoproteins and proteoglycans.

Collagen is the most important structural protein of the dermis since it represents 70 % of the dry weight of the dermis. Different, genetically distinct types of collagen exist. Among the major collagens, type I collagen is encountered in connective tissues and represents all of the collagen in bone and dentine; type III collagen is associated with type I collagen in most connective tissues, but is not found in bone or tendons. It is thus a marker for sub-epidermal connective tissue.

Many active agents have been proposed to prevent or delay the effect of ageing. It has been shown that retinoids, and in particular retinol, have a favourable effect for enhancing the appearance and state of the skin and for combating the signs of ageing.

EP-A-698392 discloses a composition of at least a retinoid with lactose and acceptable excipients.

The Applicant has now found that the combination of retinoids with melibiose makes it possible to potentiate the activity of maintaining the skin in good condition and of improving its appearance.

In the context of the present invention, it has been shown that other mechanisms for combating the effects of ageing may be potentiated. In particular, the synthesis of collagen by fibroblasts is found to be increased by melibiose; it is also increased by retinol, and a synergism of these activities exists in the presence of the two molecules.

The retinoid entering into the compositions according to the invention is preferably chosen from the group consisting of retinoic acid or tretinoin, retinol, retinaldehydes, their salts and their esters. Typical salts are the alkali metal, ammonium and C₂-C₃₀ ammonium salts. The sodium, potassium, triethanolammonium and ammonium salts are particularly preferred. Combinations of all the above compounds may be present in the compositions. In addition, the terms "retinol" and "retinoic acid" should be understood as including the hydrogenated and non-hydrogenated isomers such as 9-cis-retinol, didehydroretinol, 13-cis-retinoic acid, 13-trans-retinoic acid and didehydroretinoic acid.

Compositions which are particularly suitable for carrying out the invention contain a combination of retinol and melibiose.

They also comprise excipients suitable for external topical administration, in particular dermatologically acceptable excipients.

According to another embodiment of the invention, the compositions contain excipients suitable for oral administration.

Excipients suitable for the formulation are known to those skilled in the art and comprise in particular thickeners, emulsifiers, preserving agents, dyes, fragrances, etc..

The compositions may be, for example, in the form of solutions, gels, lotions, creams, oil-in-water, water-in-oil or multiple emulsions, or in liposome form.

The compositions may also contain other moisturizing agents or softeners.

By means of the synergistic activity of the retinoid and the oligosaccharide, in particular the combination of retinol with melibiose and/or lactose, the compositions according to the invention have an improved effect on the major signs of ageing, in terms of efficacy and speed of action. The first results may be obtained after six weeks of treatment with the compositions and are exerted deep down. These effects comprise a reduction in the number and/or depth of wrinkles, firming of the skin and better moisturization; the compositions according to the invention will also unify the complexion and/or prevent or decrease the appearance of age marks. The compositions according to the invention will be particularly suitable for combating wrinkles and slackening of the tissues. They also protect the skin against environmental attacks, in particular UV, and pollution. They give a unified appearance and prepare the skin to receive other cosmetic and make-up products.

The compositions according to the invention may be chosen to be used in the morning and/or evening on the face or the hands. On the epidermis of the hands, they will be particularly appropriate for improving the pigmentation and combating the appearance of age marks, as well as for increasing the firmness of the tissues in this region, which have a tendency to become flaccid with age.

The compositions according to the invention also allow the nails to be strengthened by preventing them from becoming brittle and allow their appearance to be improved, in particular by combating the appearance of striations and/or marks.

The compositions according to the invention are particularly appropriate for treating the areas around the eyes and the lips, which are very fragile and are highly susceptible to the appearance of wrinkles and slackening of the skin. Compositions according to the invention are very well tolerated in this sensitive area, where their anti-ageing activity, due to the synergism between the various components, will be exerted from six weeks of application onwards. They make it possible to reduce visibly the number of wrinkles and the pockets under the eyes; they firm up the particularly sensitive skin around the eyes and the mouth.

The compositions may also be applied to the exoskeleton and in particular to the hair.

Advantageously, the concentration of retinoids, in particular of retinol is greater than or equal to about 0.0001 % and less than 3 % relative to the total weight of the composition. It is preferably between 0.001 % and about 1 % by weight, and even more preferably less than or equal to about 0.5 %. Melibiose is present at a concentration of between about 0.0001 % and about 5 %; advantageously, its concentration will be greater than or equal to about 0.001 % and less than or equal to about 2 % by weight relative to the total weight of the composition, preferably less than or equal to about 1 %.

According to another aspect, the subject of the invention is the use of a combination of at least one retinoid with at least one oligosaccharide comprising from 2 to 6 monosaccharide residues and having a galactose in the non-reducing terminal position, or a derivative of such an oligosaccharide substituted with a hydrophobic residue, for the preparation of a composition designed to improve the elasticity of the skin and/or to combat the changes in the skin which are due to ageing.

More particularly, the dermocosmetic compositions according to the invention are designed to improve the viscoelastic properties of the skin and to prevent or reduce the formation of wrinkles, and this is achieved since the Applicant has demonstrated that retinol possesses an antielastase activity, which reinforces the known effect of melibiose.

By stimulating collagen synthesis, the retinol/melibiose combination leads to a densification of the collagen bundles with better organization of the bundles.

A thickening of the mucous substance in the form of epithelial expansion by invagination into the dermis is also observed.

A method for the cosmetic treatment of photo-ageing and of intrinsic ageing of the skin, the mucosae or the exoskeleton, in particular the face and the hands, which consists in applying the compositions as described above, is also included in the invention.

The examples which follow are designed to illustrate certain aspects of the invention.

### Example 1: Effect of retinol, retinoic acid, melibiose or the retinol-melibiose combination on the expression of mRNA for collagen and fibroblasts

An in vitro model based on the use of human dermal fibroblasts cultured in collagen lattices (dermal equivalent) was selected to evaluate the effects of retinol, melibiose and the retinol + melibiose combination.

The level of investigation selected was that of the messenger RNAs (mRNA) which code for procollagen III (which will be referred to hereinbelow as collagen III). The mRNAs were detected by a method of reverse transcription/heat-sensitive amplification. This technique has advantages over other methods for studying RNAs, in particular as regards the sensitivity and the possibility of quantifying (semi-quantitative) the effects.

### I - EQUIPMENT AND METHODS

### Test system

The fibroblast culture medium (FCM) consisted of MEM/199 (3/4, 1/4; v/v) supplemented with penicillin (100 IU/ml), streptomycin (100 µg/ml), glutamine (2 mM) and sodium bicarbonate (0.2 %, v/v).

The solution for washing the test system was phosphate buffered saline (PBS): 8 g/l NaCl; 1.15 g/l Na₂HPO₄; 0.2 g/l KH₂PO₄; 0.2 g/l KCl; 0.1 g/l CaCl₂; 0.1 g/l MgCl₂; pH 7.4.

The fibroblasts were isolated from a mammary plasty performed on a 34 year old woman. The cells were obtained by culturing skin explants; they were used at the seventh passage.

The fibroblasts were suspended in a solution containing collagen I (2 mg/ml), FCM medium and 5 % (v/v) FCS. The suspension was divided among 6-well culture plates and the plates were placed at 37°C, in an atmosphere containing 5 % CO₂. The test systems consisted of the lattices contracted with the fibroblasts after culturing for 96 hours.

The melibiose (M) was tested at 0.01; 0.1 and 1 µg/ml (i.e. 0.01, 0.1 and 1 ppm).

The retinol (R) was tested at 10⁻⁶, 10⁻⁵ and 10⁻⁴% (i.e. 0.01; 0.1 and 1ppm).

The retinoic acid (RA) was tested at 10⁻⁶; 10⁻⁵ and 10⁻⁴ % (i.e. 0.01; 0.1 and 1 ppm).

The combination of the M and R products was tested at 0.1 ppm + 0.01 ppm and at 0.1 ppm + 0.1 ppm respectively.

The melibiose and the retinol were dissolved directly in the MCF medium. The retinoic acid was dissolved at 0.1 % (w/v) in DMSO and then diluted in the FCM medium.

Vitamin C, taken as a positive control, was tested at 1 mM in the FCM medium.

The collagen lattices were incubated for 96 hours with the test products or the reference product, at 37°C in an atmosphere containing 5 % CO₂.

The total RNA was extracted and purified from the fibroblasts included in the collagen lattices using the Instapur RNA kit (Eurogentec, batch 17).

The integrity of the extracted RNAs and the absence of contaminating genomic DNA were verified by separation of the RNAs on a 1 % (w/v) agarose gel containing 10 µg/ml of BET.

The concentration and purity of the RNA preparations were determined by spectrophotometry at λ = 260 nm and 280 nm. The OD 260/OD 280 ratio needed to be greater than 2.

The messenger RNAs were transformed into cDNA by reverse transcription in a total volume of 20 µl of reaction mixture containing 500 ng of random primer, 0.5 mM of each dNTP, 22 U of ribonuclease inhibitor, the buffer for the enzyme and 200 U of MmLV reverse trans-criptase (Promega). This reaction was carried out at 42°C for 1 hour.

### Heat-sensitive amplification

For each mRNa studied, 2 oligonucleotides (so-called "primers") complementary to a sequence coding for the gene were chosen as a function of the following criteria:
- size of the primer, 20 nucleotides,
- distance between the primers of between 300 and 1000 base pairs,
- percentage of [guanine + cytosine] in the sequence of between 50 % and 80 %.

The characteristics of the oligonucleotides used are featured in the following table.

**Table 1:**

| Characteristics of the oligonucleotides used for the heat-sensitive amplification reaction | | | |
|---|---|---|---|
| Target mRNA (/mRNA standard coupled) | Primer hybridization temperature (°C) | Number of cycles of heat-sensitive amplification | Size of the amplified fragment (in base pairs) |
| Collagen I (/β-actin-A) | 61 | 35 | 922 |
| Collagen III (/β-actin-C) | 56 | 22 | 406 |
| Collagen VII (/β-actin-A) | 62 | 30 | 661 |
| β-actin-A | | | 300 |
| β-actin-C | | | 838 |

The target sequences of the cDNAs obtained were amplified in 20 µl of reaction mixture containing 1 mM of each of the oligonucleotides (5'→3' and 3'→5'), 0.25 mM of each nNTP, 1.5 mM of MgCl₂, the buffer for the enzyme and 0.1 U of Taq polymerase (Eurogentec).

The number of cycles which allows a reaction proportional to the initial amount of cDNA was determined for each cDNA. A cDNA was included as internal standard in each amplification, that of β-actin. Two sets of primers which differ by the optimum hybridization temperature and by the size of the amplified fragment were used for β-actin: this allowed a correct separation or reaction, for the various target sequences studied. In addition, an amplification was carried out in parallel starting with non-reverse-transcript RNAs and a genomic DNA, as complementary controls, in each experimental series. Each amplification reaction was carried out in duplicate.

The products obtained from the heat-sensitive amplification (referred to as amplicons) were separated by electrophoresis on 2 % (w/v) agarose gel containing 10 µg/ml of BET, a dye for the amplicons. The intensity of the bands corresponding to the amplicons studied was measured using a densimeter scanner coupled to the Densylab programme). The results were expressed as a band intensity and also in the form of a ratio between the intensity of the band for the amplicon obtained from the standard mRNA.

### 2 - Results

Expression of the target mRNAs was studied by a technique of reverse transcription-heat sensitive amplification. Several controls were carried out at each step in order to be able to compare and (semi)quantify the levels of expression of the target mRNAs.

These controls were, in particular, the following:
- quality of the RNAs extracted,
- absence of genomic DNA contamination,
- specificity of the primers,
- linearity of the amplification reaction,
- inclusion of an internal standard.

The results were expressed in terms of a target mRNA/standard mRNA ratio. Differences higher than a factor 1.5 were considered as significant.
Collagen III

The ratio of intensities observed for the fibroblasts cultured in the presence of the reference product (vitamin C), tested at 1 mM, was higher than that of the control by a factor of 2.6 (Table 2).

The ratio of intensities observed for the fibroblasts cultured in the presence of melibiose (M), tested at 0.01 ppm, was higher than that of the control by a factor of 1.5. At the other test concentrations, the intensity ratios obtained were comparable with that of the control (Table 2).

The intensity ratios observed for the fibroblasts cultured in the presence of retinol (R), tested at 0.01 and 0.1 ppm, were higher than that of the control by a factor of 1.5 and 1.9 respectively (Table 2).

The ratio of intensities observed for the fibroblasts cultured in the presence of retinoic acid (RA), tested at 0.01 ppm, was higher than that of the control by a factor of 2.4 (Table 2).

The intensity ratios observed for the fibroblasts cultured in the presence of the product M+R, tested at 0.1 ppm + 0.01 ppm and 0.1 ppm + 0.1 ppm, were higher than that of the control by a factor of 3.4 and 2.7 respectively (Table 2).

Comparison of the target mRNA/standard mRNA densimetric intensity ratios demonstrates an effect of the M, R, RA and M+R test products on the expression of the mRNAs coding for collagen III by human dermal fibroblasts cultured on a collagen lattice.

The test products increase the expression of the mRNAs coding for collagen III. The intensity of this effect was variable depending on the products. The largest effect was observed with the M+R product, followed by the RA and R product. The M, RA and M+R products exhibited a dose effect relationship; the most significant effects were observed at the lowest concentrations tested.

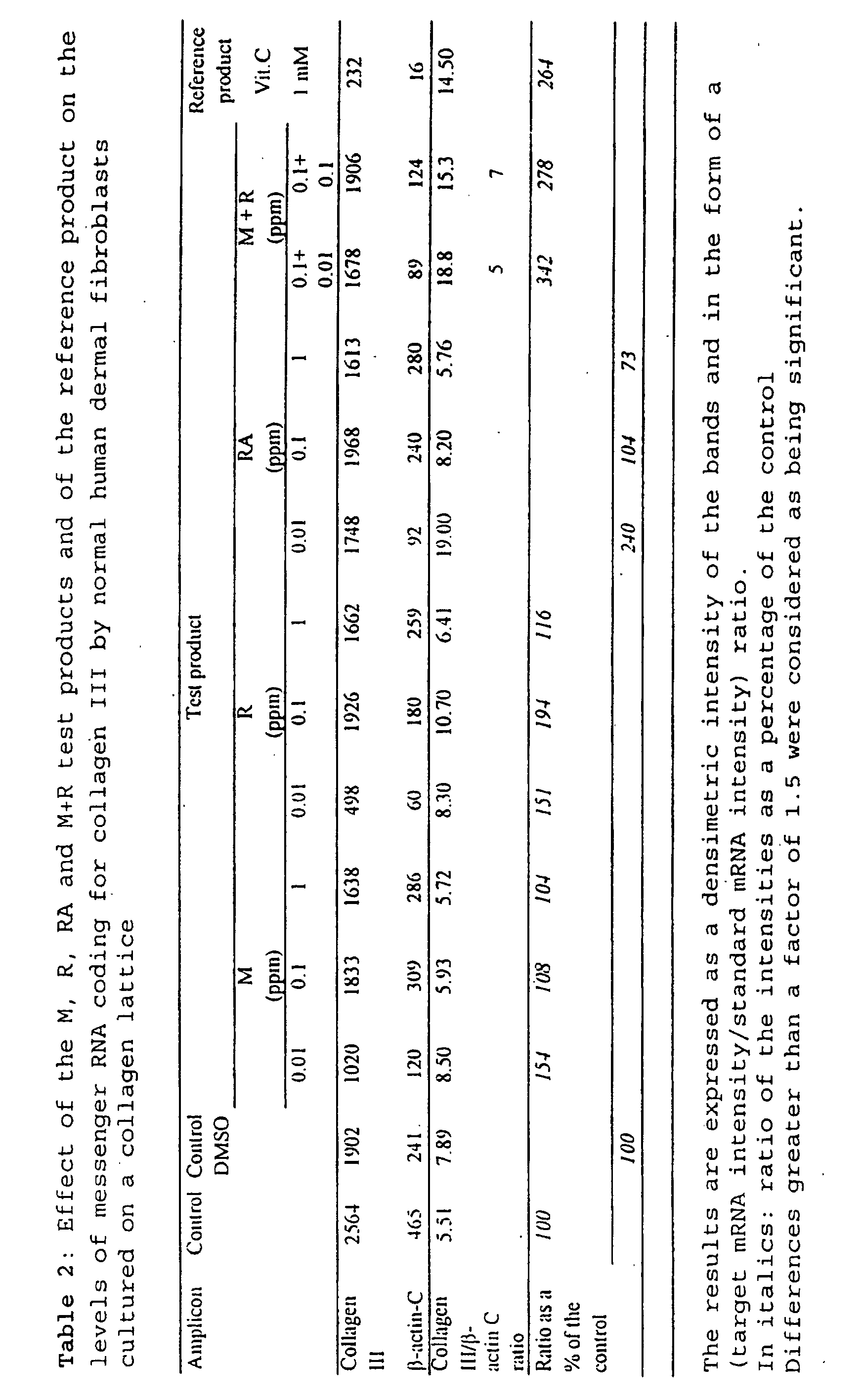

### Example 2: Effect on human skin of retinol alone or in combination with melibiose

The effects of three cosmetic creams on normal human skin are analysed after 28 days of application.

For this, a model of human skin kept alive by organ culture is used.

### 1 - Equipment and methods

The organ cultures are carried out according to the following procedure. In a first stage, the skin fragments (source: plastic surgery) are placed in inserts which are themselves positioned on culture wells. The three products are placed directly on the skin. Culture medium (antibiotic, FCS) is added to the bottom of the wells, one passage being made by slow diffusion between the two compartments via a porous membrane (0.45 µm).

The assembly is maintained under organ culture conditions for 28 days. The culture medium in the wells is also renewed three times a week. The three creams are renewed three times a week on the skins:
- cream 1: retinol
- cream 2: retinol + melibiose
- cream 3: excipient

Keeping an untreated skin alive for 28 days allows a control of culture manipulation.

### 1-1- Study of the epithelium and of the collagen

**a) Analysis of the epithelium**
   - by a histological analysis after fixing in Bouin liquid and inclusion in paraffin with staining using eosin-haemalum which allows:
   - calculation of the number of layers in the mucous substance and the thickness of the horny layer;
   - by an immunohistochemical analysis of the epithelial differentiation using antibodies raised against total cytokeratins.
**b) Study of the collagen**
   - by an histological analysis after staining with Masson green trichrome stain.
   - gel electrophoresis analysis of type I and III collagens

Another series of skins is collected and the collagen is extracted after treatment with pepsin. Gel electrophoresis (SDS-PAGE with 7.5 % acrylamide) with reduction using β-mercaptoethanol makes it possible to isolate and quantify the specific collagen chains of types I and III as a function of their molecular weight relative to the other skin proteins (technique of Sykes et al.).

### 1-2- Analysis of the antielastase activity by studying the protection of elastic fibres in human skins kept alive ex vivo

Using the human skin model on which an experimental procedure of destruction of the elastic fibre network by elastases from polymorphonuclear leucocytes and from macrophages is used.

For this, human leucocyte elastase (HLE) is added to the surface of the skin three times a week, creams optionally containing an active agent for antielastase purposes being placed on the epidermis at the same time three times a week.

At the end of 28 days of culturing, the skin fragments are fixed in Bouin liquid and the elastic fibres are revealed by staining with (+)-catechin. The conservation of the elastic fibres is analysed:
- histologically,
- by quantification by image analysis of the surface of the elastic fibres.

### 2- RESULTS

### 2.1- STUDY OF THE EPITHELIUM BY HISTOLOGICAL AND IMMUNOHISTOCHEMICAL TECHNIQUES

### a) Histological analysis of the thickness of the epithelium and of the horny layer

The number of layers after ex vivo culturing for 28 days was calculated for the mucous substance, as well as the thickness of the horny layer (average ± SD) using histological slices stained with haemalum-eosin (HE) . The minimum and maximum number of layers (dermal expansions) of the mucous substance were counted.

| Number of layers in the epidermis | | | |
|---|---|---|---|
| Horny layer | | Mucous substance | |
| | | min. number of layers | expansion |
| skin treated with cream 1 | 5.95 ± 2.55 | 7 ± 2.9^{*} | 7 ± 0.7^{*} |
| skin treated with cream 2 | 6.65 ± 3.1 | 5.3 ± 1.5 | 8.36 ± 1.65^{*} |
| skin treated with cream 3 | 7.6 ± 2.9 | 3.9 ± 0.9 | 0 |

| | | | |
|---|---|---|---|
| ^{*} result statistically different to that of the excipient, the cream No. 3 (p < 0.05; Student test) | | | |

### b) Results regarding the horny layer:

The skins treated with creams 1, 2 and 3 show no significant changes in the thickness of the horny layer.

### Results regarding the mucous substance:

In the case of the skins treated with creams 1 and 2, the presence of a thickening of the mucous substance in the form of epithelial expansion relative to cream 3 (excipient) is noted.

The expansion is longer for cream No. 2 (retinol + melibiose).

### c) Immunohistochemical analysis of the epithelial differentiation using antibodies raised against total cytokeratins

With the skins treated with cream 2, an increase in the intensity of the labelling (reflecting an improvement in the differentiation) of substantially identical appearance is observed in 6 out of 8 cases.

### 2.2- HISTOLOGICAL AND BIOCHEMICAL ANALYSIS OF THE COLLAGEN

### a) Histological analysis (staining with the Masson trichrome stain)

For each cream, an evaluation by optical microscope of the intensity of the labelling and of the organization of the collagen bundles is made comparatively.

The following is observed in all cases:
- cream No. 2 > cream No. 1 > cream No. 3.

Densification of the collagen bundles with an increase in the thickness of the bundles and their number, thus reducing the intercellular spaces is clearly observed with cream No. 2; this phenomenon is especially visible in the upper dermis in contact with the basal membrane. This modification in the organization of the bundles is responsible for the increase in the intensity of the labelling with the trichrome stain. The bundles also appear to be better organized.

### b) Analsis by gel electrophoresis of type I and III collagens

Gel electrophoresis made it possible to demonstrate an increase in the synthesis of collagen for the skins treated with creams 1 and 2, since the protein bands revealed corresponding to the type I and III collagens have a higher labelling intensity than that of cream 3. It should be noted that the increase in synthesis appears higher for the type I collagen than for the type III collagen. The amount of collagen (type I and II) in the skins treated with cream 3 is no different to that in a normal skin.

### 2-3 EVALUATION OF ADVERSE CHANGES IN THE ELASTIC FIBRES INDUCED BY HLE IN SKINS KEPT ALIVE AND EVALUATION OF THEIR PROTECTION AFTER TREATMENT WITH THREE CREAMS HAVING ANTI-AGEING FUNCTIONS

### a) Histological analysis of the elastic fibres

On the 6 skins analysed, the experimental procedure of destroying the elastic fibres using HLE allowed partial degradation of the elastic fibre network to be obtained. This destruction is variable from one skin to another.

Thus, comparison of the percentage protection of the elastic fibres will be carried out between the biopsy treated with HLE and that treated with cream. Secondly, an average of the percentages in taken on the 6 skins.

For the skins treated with cream No. 1, the protection, evaluated semi-quantitatively, is equal to 40 %. For the skins treated with cream No. 2, the protection obtained is 41.25 %. Lastly, for those treated with cream No. 3, the protection is 0 %; the destruction of the elastic fibres is identical to that obtained with human leucocyte elastase alone.

### b) Quantification by image analysis of the elastic fibres

A quantitative analysis of the elastic network of the dermis was carried out on the skins treated with the HLE enzyme in the presence or absence of the four test creams.

The percentage area of dermis occupied by the elastic fibres stained with (+)-catechin was measured.

The results obtained on average for the 6 cases analysed are:

| | % of surface area |
|---|---|
| control skins | 12.6 % ± 0.6 |
| skins + HLE | 4 % ± 0.5 |
| | |
| skins treated with cream 1 + HLE | 7.5 % ± 0.5^{*} |
| skins treated with cream 2 + HLE | 7.8 % ± 0.7^{*} |
| skins treated with cream 3 + HLE | 4.2 % ± 0.2 |

| | |
|---|---|
| ^{*} result statistically different to that of the skin + HLE (p < 0.05; Student test) | |

Creams 1 and 2 made it possible to protect the dermal elastic tissue against destruction by human leucocyte elastase. On the other hand, no protection was obtained with cream No. 3.

Evaluation of the size of the elastic fibres makes it possible to measure the average of the longest (µm) elastic fibres on several areas.

| | µm |
|---|---|
| control skins | 58 ± 5 |
| skins + HLE | 36 ± 8.6 |
| | |
| skins treated with cream 1 + HLE | 54 ± 4.7^{*} |
| skins treated with cream 2 + HLE | 54 ± 5.4^{*} |
| skins treated with cream 3 + HLE | 35 ± 7.7 |

| | |
|---|---|
| ^{*} result statistically different to that of the skin / HLE (p < 0.05; Student test) | |

The skins treated with creams 1 and 2 have fibres similar in length to the control skins for the longest fibres.

After treatment with HLE, the fragmented appearance histologically observed for the elastic fibres is confirmed by image analysis: the elastic fibres have a maximum length of 36 µm, this result being significantly different from that of the control skins (p < 0.05; Student test).

The skins treated with cream 3 did not allow the elastic fibres to be protected against fragmentation induced by human leucocyte elastase. The length obtained is, in fact, 35 µm at most for the longest fibres.

### Example 3: Influence of retinol and melibiose on the antielastase activity on slices of normal human skin Equipment and methods

Frozen slices 8 µm in thickness were made from samples obtained in plastic surgery. The human leucocyte elastase (HLE at 10 µg/ml) was applied to the skin slices for 2 hours at room temperature and in a humid atmosphere with or without retinol or melibiose. Thus, the elastic fibres were subjected directly to the enzymatic action.

After fixing in acetone and dehydration in ethanol at 70°C, the elastic fibres which remained after treatment were stained with (+)-catechin. Semiquantitative evaluation of the elastic fibres was completed by quantification by means of image analysis.

The antielastase activity of the retinol and melibiose was compared with that of PMSF (phenylmethylsulphonyl fluoride), a powerful elastase inhibitor.

### Results

The experimental results are collated in the following table:

| Products | | % antielastase activity |
|---|---|---|
| Retinol | 1 mg/ml | 80 % |
| | 100 µg/ml | 70 % |
| | 10 µg/ml | 60 % |
| Melibiose | 1 mg/ml | 15 % |

The retinol thus has considerable antielastase activity. This effect is dose-dependent.

The melibiose also has antielastase activity, but this effect is not dose-dependent.

### Example 4: Compositions containing combinations according to the invention

| **Formula A: O/W emulsion** | |
|---|---|
| | % |
| - glycerol | 5 |
| - disodium EDTA | 0.2 |
| - methyl paraben/phen oxyethanol/propyl paraben | 1 |
| - water | 65.3 |
| - polyacrylamide and C13-14 isoparaffin and laureth-7 | 2 |
| - dimethicone | 2 |
| - cetearyl alcohol | 0.5 |
| - cyclomethicone | 1.5 |
| - isopropyl palmitate | 2 |
| - BHF | 0.1 |
| - tri-C14-C15 alkyl citrate | 4 |
| - 90° alcohol | 6 |
| - caffeine | 2 |
| - melibiose | 0.5 |
| - retinol | 0.3 |
| - butylene glycol | 2 |
| - lactose | 4.5 |
| - stearic acid | 1.0 |
| - sodium hydroxide | 0.1 |

| **Formula B: O/W emulsion** | |
|---|---|
| | % |
| - Water | 67.473 |
| - Carbomer | 0.300 |
| - Glycerol | 5.000 |
| - PEG 8 | 2.000 |
| - Disodium EDTA | 0.200 |
| - Sodium hydroxide | 0.135 |
| - Water | 1.000 |
| - Melibiose | 0.500 |
| - Lactose | 4.50 |
| - Phenoxyethanol/methyl paraben/propyl paraben | 0.200 |
| - Glyceryl stearate/Peg 100 stearate | 5.000 |
| - Cetearyl octanoate/isopropyl myristate | 3.000 |
| - C12-15 alkyl benzoate | 3.500 |
| - Glyceryl stearate | 3.000 |
| - stearyl alcohol | 0.500 |
| - Cetostearyl alcohol | 0.500 |
| - Cetyl palmitate | 0.500 |
| - Talc | 0.200 |
| - BHT | 0.100 |
| - Phenoxyethanol/methyl paraben/propyl paraben | 0.800 |
| - C12-15 alkyl benzoate | 0.500 |
| - Retinol | 0.092 |
| - Dimethicone | 1.000 |
| Total | 100.- |

| **Formula C: O/W emulsion** | |
|---|---|
| | % |
| - Water | 64.808 |
| - Carbomer | 0.100 |
| - Crosslinked acrylate/ crosslinked C10-C30 alkyl acrylate polymer | 0.500 |
| - Poloxamer 407 | 0.500 |
| - Glycerol | 5.000 |
| - Polyglyceryl methacrylate/aqueous propylene glycol | 5.000 |
| - Disodium EDTA | 0.200 |
| - Sodium hydroxide | 0.200 |
| - Water | 1.000 |
| - Melibiose | 0.500 |
| - Lactose | 4.50 |
| - Phenoxyethanol/methyl paraben/propyl paraben | 0.200 |
| - Cetearyl octanoate/ isopropyl myristate | 5.000 |
| - Octyl methoxycinna mate | 8.000 |
| - Butyl methoxydiben zoylmethane | 1.500 |
| - Talc | 1.000 |
| - BHT | 0.100 |
| - Phenoxyethanol/methyl paraben/propyl paraben | 0.800 |
| - Tocopheryl acetate | 0.500 |
| - Cetearyl octanoate/ isopropyl myristate | 0.500 |
| - Retinol | 0.092 |
| Total | 100.- |

| **Formula D: O/W emulsion** | |
|---|---|
| | % |
| - Xanthan gum | 0.100 |
| - Water | 57.802 |
| - Glycerol | 3.000 |
| - PEG 8 | 5.000 |
| - Disodium EDTA | 0.200 |
| - Lactose | 5.000 |
| - Polyglyceryl methacrylate/aqueous propylene glycol | 5.000 |
| - Glycine | 0.200 |
| - Melibiose | 0.500 |
| - Urea | 2.000 |
| - Phenoxyethanol/methyl paraben/propyl paraben | 0.200 |
| - Cetearyl alcohol, cetearyl glucoside | 5.000 |
| - Isocetyl stearate | 4.500 |
| - Trioactanoine | 2.000 |
| - Di(C12-C13)alkyl malate | 5.000 |
| - Dimethicone | 1.000 |
| - Cyclomethicone | 2.000 |
| - BHT | 0.100 |
| - Phenoxyethanol/methyl paraben/propyl paraben | 0.800 |
| - Isocetyl stearate | 0.500 |
| - Retinol | 0.092 |

| **Formula E: Gel** | |
|---|---|
| | % |
| - Glycerol | 5 |
| - Disodium EDTA | 0.2 |
| - Methyl paraben/ phenoxyethanol/propyl paraben | 1 |
| - Water | 66.4 |
| - Polyacrylamide and C13-14 isoparaffin and laureth-7 | 2 |
| - Dimethicone | 2 |
| - Cetearyl alcohol | 0.5 |
| - Cyclomethicone | 1.5 |
| - Isopropyl palmitate | 2 |
| - BHT | 0.1 |
| - Alkyl tri-C14-C15-citrate | 4 |
| - 90° alcohol | 6 |
| - Caffeine | 2 |
| - Melibiose | 0.5 |
| - Retinol | 0.3 |
| - Butylene glycol | 2 |
| - Lactose | 4.5 |

| **Formula F: O/W emulsion** | |
|---|---|
| | % |
| - Glycerol | 5 |
| - Disodium EDTA | 0.2 |
| - Methyl paraben/ phenoxyethanol/propyl paraben | 1 |
| - Water | 65.4 |
| - Polyacrylamide and C13-14 isoparaffin and laureth-7 | 2 |
| - Dimethicone | 2 |
| - Cetearyl alcohol | 0.5 |
| - Cyclomethicone | 1.5 |
| - Isopropyl palmitate | 2 |
| - BHT | 0.1 |
| - Alkyl tri-C14-C15- | 4 |
| citrate | |
| - 90° alcohol | 6 |
| - Caffeine | 2 |
| - Melibiose | 0.5 |
| - Retinol | 0.3 |
| - Butylene glycol | 2 |
| - Lactose | 4.5 |
| - Stearic acid | 1.0 |
| - Sodium hydroxide | 0.1 |
| - Cetearyl alcohol/ cetearyl glucoside | 1.0 |

| **Formula G: O/W emulsion** | |
|---|---|
| | % |
| - Water | 60.491 |
| - Carbomer | 0.300 |
| - Water | 1.000 |
| - Sodium hydroxide | 0.135 |
| - Chondrus crispus | 1.000 |
| - Magnesium aluminium silicate | 0.250 |
| - Disodium EDTA | 0.200 |
| - Lactose | 4.000 |
| - Kaolin | 1.000 |
| - PEG 8 | 1.000 |
| - Glycyrrhetinic acid | 0.500 |
| - Melibiose | 1.00 |
| - Sorbitol | 2.000 |
| - Phenoxyethanol/methyl paraben/propyl paraben | 0.200 |
| - Isocetyl stearate | 5.500 |
| - Cetearyl octanoate/ isopropyl myristate | 4.000 |
| - Sorbitan stearate | 4.080 |
| - PEG 20 stearate | 7.920 |
| - Cetearyl alcohol | 2.000 |
| - BHT | 0.100 |
| - Phenoxyethanol/methyl paraben/propyl paraben | 0.800 |
| - Isocetyl stearate | 0.500 |
| - Retinol | 0.024 |
| - Hamamelis virginiana | 2.000 |

## Claims

1. Pharmaceutical or cosmetological composition, **characterized in that** it contains a combination of at least one retinoid with melibiose, or a derivative of such melibiose substituted with a hydrophobic residue, or a mixture of melibiose or melibiose derivative with lactose,
and pharmaceutically or cosmetologically acceptable excipients.

2. Pharmaceutical or cosmetological composition according to Claim 1, **characterized in that** the retinoid is chosen from the group consisting of retinol, retinoic acid, their salts and their esters.

3. Pharmaceutical or cosmetological composition according to one of Claims 1 or 2, **characterized in that** it contains a combination of retinol and melibiose.

4. Pharmaceutical composition according to one of Claims 1 to 3, **characterized in that** it contains dermatologically acceptable excipients.

5. Pharmaceutical composition according to one of Claims 1 to 3, **characterized in that** it also contains excipients suitable for oral administration.

6. Composition according to one of Claims 1 to 5, **characterized in that** the retinoid is present at a concentration of between 0.0001 and 3% by weight, in combination with melibiose in a concentration of between 0.0001 and 5 % by weight, relative to the total weight of the composition.

7. Composition according to one of Claims 1 to 5, **characterized in that** the retinol is present at a concentration of between 0.0001 % and 3 %, and **in that** the melibiose is present at a concentration of between 0.001 % and 2 %.

8. Use of a combination of at least one retinoid with melibiose or a derivative of melibiose substituted with a hydrophobic residue, for the preparation of a composition designed to improve the elasticity of the skin and/or to prevent or treat changes in the skin and the exoskeleton which are due to ageing.

9. Use of a combination according to Claim 8, **characterized in that** the retinoid is retinol.

10. Use according to one of Claims 8 and 9, for the preparation of a dermocosmetic composition designed to improve the viscoelastic properties of the skin and to prevent or reduce the formation of wrinkles.

11. Method for the cosmectic treatment of the changes associated with ageing of the skin and/or the exoskeleton, **characterized in that** a composition according to one of Claims 1 to 3, 6 and 7 is applied to the skin and/or the exoskeleton.

## Patentansprüche

1. Pharmazeutische oder kosmetische Zusammensetzung, **dadurch gekennzeichnet, daß** sie eine Kombination von mindestens einem Retinoid mit Melibiose oder einem Derivat von Melibiose, das mit einem hydrophoben Rest substituiert ist, oder einer Mischung von Melibiose oder einem Melibiose-Derivat mit Lactose und pharmazeutisch oder kosmetisch annehmbare Träger enthält.

2. Pharmazeutische oder kosmetische Zusammensetzung nach Anspruch 1, **dadurch gekennzeichnet, daß** das Retinoid ausgewählt ist aus der Gruppe bestehend aus Retinol, Retinoesäure, deren Salzen und deren Estern.

3. Pharmazeutische oder kosmetische Zusammensetzung nach Anspruch 1 oder 2, **dadurch gekennzeichnet, daß** sie eine Kombination von Retinol und Melibiose enthält.

4. Pharmazeutische Zusammensetzung nach einem der Ansprüche 1 bis 3, **dadurch gekennzeichnet, daß** sie dermatologisch annehmbare Träger enthält.

5. Pharmazeutische Zusammensetzung nach einem der Ansprüche 1 bis 3, **dadurch gekennzeichnet, daß** sie auch einen Träger enthält, der für eine orale Verabreichung geeignet ist.

6. Zusammensetzung nach einem der Ansprüche 1 bis 5, **dadurch gekennzeichnet, daß** das Retinoid bei einer Konzentration zwischen 0,0001 und 3 Gew.-% in Kombination mit Melibiose in einer Konzentration zwischen 0,0001 und 5 Gew.-%, bezogen auf das Gesamtgewicht der Zusammensetzung, vorliegt.

7. Zusammensetzung nach einem der Ansprüche 1 bis 5, **dadurch gekennzeichnet, daß** das Retinol bei einer Konzentration zwischen 0,0001% und 3% vorliegt und daß die Melibiose bei einer Konzentration zwischen 0,001% und 2% vorliegt.

8. Verwendung einer Kombination von mindestens einem Retinoid mit Melibiose oder einem Derivat von Melibiose, das mit einem hydrophoben Rest substituiert ist, für die Herstellung einer Zusammensetzung, die dazu bestimmt ist, die Elastizität der Haut zu verbessern und/oder Änderungen der Haut und des Exoskeletts, die auf Alterung beruhen, zu verhüten oder zu behandeln.

9. Verwendung einer Kombination nach Anspruch 8, **dadurch gekennzeichnet, daß** das Retinoid Retinol ist.

10. Verwendung nach einem der Ansprüche 8 und 9 für die Herstellung einer dermokosmetischen Zusammensetzung, die dazu bestimmt ist, die viskoelastischen Eigenschaften der Haut zu verbessern und die Bildung von Falten zu verhüten oder zu verringern.

11. Verfahren zur kosmetischen Behandlung der Änderungen, die mit der Alterung der Haut und/oder des Exoskeletts verbunden sind, **dadurch gekennzeichnet, daß** eine Zusammensetzung nach einem der Ansprüche 1 bis 3, 6 und 7 auf der Haut und/oder dem Exoskelett aufgetragen wird.

## Revendications

1. Composition pharmaceutique ou cosmétologique, **caractérisée en ce qu'**elle contient une combinaison d'au moins un rétinoide et de mélibiose, ou d'un dérivé de ce mélibiose substitué par un résidu hydrophobe, ou d'un mélange de mélibiose ou d'un dérivé de mélibiose avec du lactose, et des excipients acceptables d'un point de vue pharmaceutique ou cosmétologique.

2. Composition pharmaceutique ou cosmétologique selon la revendication 1, **caractérisée en ce que** le rétinoïde est choisi dans l'ensemble comprenant le rétinol, l'acide rétinoïque, leurs sels et esters.

3. Composition pharmaceutique ou cosmétologique selon l'une des revendications 1 ou 2, **caractérisée en ce qu'**elle contient une combinaison de rétinol et de mélibiose.

4. Composition pharmaceutique selon l'une des revendications 1 à 3, **caractérisée en ce qu'**elle contient des excipients acceptables d'un point de vue dermatologique.

5. Composition pharmaceutique selon l'une des revendications 1 à 3, **caractérisée en ce qu'**elle contient des excipients convenant à une administration orale.

6. Composition selon l'une des revendications 1 à 5, **caractérisée en ce que** le rétinoide est présent à une concentration comprise entre 0,0001 et 3 % en poids, en combinaison avec du mélibiose à une concentration comprise entre 0,0001 et 5 % en poids, par rapport au poids total de la composition.

7. Composition selon l'une des revendications 1 à 5, **caractérisée en ce que** le rétinol est présent à une concentration comprise entre 0,0001 et 3 %, et **en ce que** le mélibiose est présent à une concentration comprise entre 0,001 et 2 %.

8. Utilisation d'une combinaison d'au moins un rétinoïde et de mélibiose ou d'un dérivé de mélibiose substitué par un résidu hydrophobe, pour préparer une composition destinée à améliorer l'élasticité de la peau et/ou à prévenir ou traiter les changements dans la peau et l'exosquelette qui sont dus au vieillissement.

9. Utilisation d'une combinaison selon la revendication 8, **caractérisée en ce que** la rétinoïde est le rétinol.

10. Utilisation selon l'une des revendications 8 et 9 pour préparer une composition dermo-cosmétique destinée à améliorer les propriétés viscoélastiques de la peau et à prévenir ou réduire la formation de rides.

11. Procédé de traitement cosmétique des changements associés au vieillissement de la peau et/ou de l'exosquelette, **caractérisé en ce qu'**on applique sur la peau et/ou l'exosquelette une composition selon l'une des revendications 1 à 3, 6 et 7.
